# EUROPEAN PATENT SPECIFICATION

(11) **EP 4 134 067 B1**
(45) Date of publication and mention of the grant of the patent: **09.07.2025**
(21) Application number: 21783999.2
(22) Date of filing: 26.03.2021
(51) Int. Cl.: A61K 8/39, A61K 8/02, A61K 8/34, A61K 8/86, A61Q 19/00

(54) **LAMELLAR GEL-CONTAINING COMPOSITION, EMULSIFIED COMPOSITION, AND COMPOSITION FOR AGENT FOR EXTERNAL USE ON SKIN**
LAMELLARES GEL ENTHALTENDE ZUSAMMENSETZUNG, EMULGIERTE ZUSAMMENSETZUNG UND ZUSAMMENSETZUNG FÜR MITTEL ZUR ÄUSSERLICHEN ANWENDUNG AUF DER HAUT
COMPOSITION CONTENANT UN GEL LAMELLAIRE, COMPOSITION ÉMULSIFIÉE ET COMPOSITION POUR UN AGENT POUR UNE UTILISATION EXTERNE SUR LA PEAU

(30) Priority: 06.04.2020 JP 2020068416
(43) Date of publication of application: 15.02.2023
(73) Proprietor: Shiseido Company, Ltd., Chuo-ku Tokyo 104-0061 (JP)
(72) Inventor: MIYAHARA, Reiji, Tokyo 104-0061 (JP); MUNAKATA, Hidehito, Tokyo 104-0061 (JP); TAKAHASHI, Shigeo, Tokyo 104-0061 (JP); ZHANG, Yang, Tokyo 104-0061 (JP)
(74) Representative: Ter Meer Steinmeister & Partner
(86) International application number: PCT/JP2021/012808
(87) International publication number: WO 2021/205902

(56) References cited:
- EP-A1- 3 409 264
- EP-A1- 3 417 845
- JP-A- 2000 001 423
- JP-A- 2017 132 765
- US-A1- 2019 350 833
- SUZUKI, TOSHIYUKI: "Progress in emulsion technology, particularly gel-emulsions", FRAGRANCE JOURNAL, vol. 2014, no. 10, 30 September 2014 (2014-09-30), JP , pages 12 - 20, XP009540033, ISSN: 0288-9803
- ARAMAKI, KENJI; KUNIEDA, HIRONOBU: "Phase Behavior of Surfactant Solutions and Organic Structure Thereof.", FRAGRANCE JOURNAL, no. Special Edition 19. Advances in Emulsification Tec, 1 April 2005 (2005-04-01), JP , pages 2 - 9, XP009540032, ISSN: 0289-1840

## Description

### Cross Reference to Related Applications

The present application is based upon and claims the benefit of priority from Japanese Patent Application No. 2020-068416 filed on April 6, 2020.

### Technical Field

The present disclosure relates to a composition including a lamellar gel phase. The present disclosure also relates to an emulsified composition employing a lamellar gel phase. The present disclosure also relates to a composition for an external-use skin preparation including a lamellar gel phase.

### Background Art

Conventionally, lamellar gel-containing external-use skin preparations employing a lamellar gel (α-gel) formed by higher aliphatic alcohols/higher fatty acids and hydrophilic surfactants have been used with the aim of maintaining emulsion stability of external-use skin preparations such as cosmetic products, quasi-pharmaceutical products, pharmaceutical products, etc. (see, for example, Patent Literature 1 and Non-Patent Literature 1).

Patent Literature 1 discloses an α-gel forming composition including (A) 25 to 50% by mass of at least one type of component selected from higher aliphatic alcohols and/or higher fatty acids having at least 16 carbon atoms, (B) 40 to 70% by mass of a polyoxyethylene sterol ether represented by a specific formula, and (C) 5 to 20% by mass of polyoxyethylene dialkyl ester(s) and/or ether(s) represented by a specific formula, wherein the composition is produced by adding water. EP3409264A1 and US2019/350833 A1 also disclose similar compositions.

### Citation List

### Patent Literature

Patent Literature 1: Japanese Unexamined Patent Publication No. 2017-132709A
Non-Patent Literature 1: Kei Watanabe et al., J. Oleo Sci., 61, 29-34 (2012)

### Summary of Invention

### Technical Problem

The following analysis can be made from the perspective of the present disclosure.

Lamellar gel-containing compositions containing higher aliphatic alcohols or higher fatty acids, such as the composition disclosed in Patent Literature 1, tend to cause a slimy feel when being applied to the skin, and hence, a user cannot obtain a refreshing feel upon use. Such lamellar gel-containing compositions also have a drawback in that, over time, the higher alcohols and higher fatty acids are likely to precipitate out as crystals (see, for example, Non-Patent Literature 1).

Further, α-gels formed by higher aliphatic alcohols, such as the composition disclosed in Patent Literature 1, generally exhibit high solubility with respect to oily components with high polarity. Hence, such α-gel-forming compositions, as those disclosed in Patent Literature 1, are not suitable as emulsifiers for highly polar oily components.

There has thus been a demand for a composition capable of offering a refreshing feel upon use while exhibiting excellent emulsion stability even with respect to highly polar oily components.

### Solution to Problem

According to a first aspect of the present disclosure, a composition according to present claim 1 including a lamellar gel phase is provided. The composition contains a first nonionic surfactant represented by a formula shown in Chem. 1 below. A content by percentage of higher aliphatic alcohols or higher fatty acids is 1% by mass or less relative to the mass of the composition.

In the formula shown in Chem. 1, R¹ is a C₁₆₋₂₄ linear acyl group or linear alkyl group. R² is a C₂₋₄ alkylene group. R³ is a C₁₆₋₂₄ linear acyl group or linear alkyl group. k represents an integer from 4 to 15.

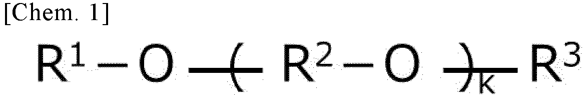

According to a second aspect of the present disclosure, an emulsified composition is provided, the emulsified composition containing the composition according to the first aspect, an oily component, and water. The oily component or the water is emulsified by the lamellar gel phase.

According to a third aspect of the present disclosure, a composition for an external-use skin preparation containing the composition(s) according to the first aspect and/or the second aspect is provided.

### Advantageous Effects of Invention

The composition of the present disclosure can offer a refreshing feel upon use when applied to the skin.

The lamellar gel phase of the present disclosure has low solubility with respect to polar oils. The lamellar gel-containing composition of the present disclosure can stably emulsify even polar oils.

### Brief Description of Drawings

[FIG. 1] FIG. 1 shows small angle/wide angle X-ray scattering charts and differential calorimetry charts in Test Example 1 (reference example)
[FIG. 2] FIG. 2 shows small angle/wide angle X-ray scattering charts and differential calorimetry charts in Test Example 2.
[FIG. 3] FIG. 3 shows small angle/wide angle X-ray scattering charts and differential calorimetry charts in Test Example 3.
[FIG. 4] FIG. 4 shows small angle/wide angle X-ray scattering charts and differential calorimetry charts in Test Example 4.
[FIG. 5] FIG. 5 shows a graph comparing the enthalpy of fusion in Test Examples 1 to 4.
[FIG. 6] FIG. 6 shows photographs of an oily component solubility test in Test Example 5.
[FIG. 7] FIG. 7 shows results of a usability test in Test Example 7.
[FIG. 8] FIG. 7 shows results of a usability test in Test Example 7. Description of Embodiments

Preferred modes of the aforementioned aspects will be described below.

According to a prefered mode of the aforementioned first aspect, the first nonionic surfactant is polyethylene glycol distearate in which k shown in the formula of Chem. 1 is from 4 to 8.

According to a prefered mode of the aforementioned first aspect, a content of the first nonionic surfactant is from 0.1 to 40% by mass relative to the mass of the composition.

According to the aforementioned first aspect, the composition further comprises a second nonionic surfactant having an HLB of from 7 to 15, and water. The first nonionic surfactant, the second nonionic surfactant, and the water constitute at least a portion of the lamellar gel phase.

According to the aforementioned first aspect, the second nonionic surfactant is at least one selected from the group consisting of compounds represented by formulas shown in Chem. 3 to Chem. 5 below.

In the formula shown in Chem. 3, R⁶, R¹⁰ and R¹⁴ are each a C₂₋₄ alkylene group. R⁷, R¹¹ and R¹⁵ are each a C₈₋₁₂ alkylene group. R⁸, R¹² and R¹⁶ are each a C₄₋₈ alkyl group. R⁹, R¹³ and R¹⁷ are each a polymer of 12-hydroxystearic acid or polymer of an alkylene polyol. m, n and o are each a natural number. The sum of m, n and o is from 10 to 60.

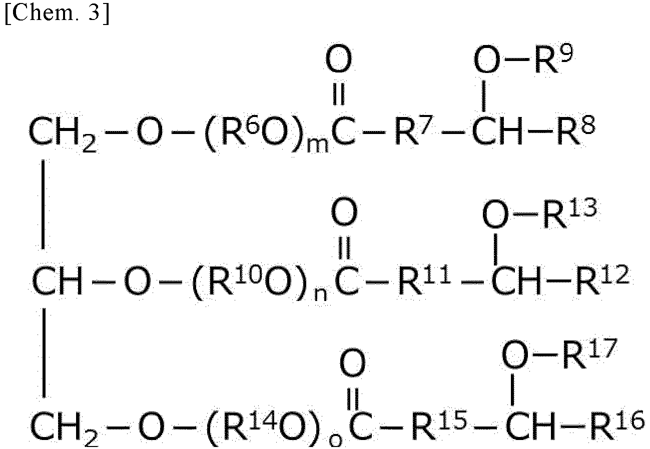

In the formula shown in Chem. 4, R¹⁸ is a C₁₆₋₂₄ linear acyl group or linear alkyl group. R¹⁹ and R²⁰ are each a C₂₋₄ alkylene group. p and q are each a natural number. The sum of p and q is from 5 to 20.

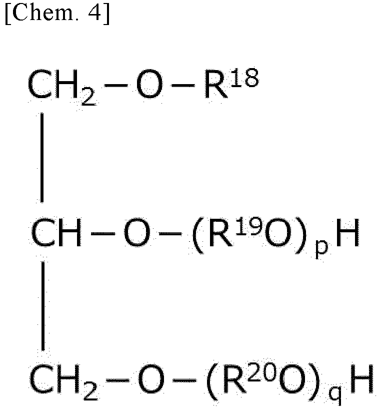

In the formula shown in Chem. 5, R²¹, R²², R²³ and R²⁵ are each a C₂₋₄ alkylene group. r, s, t and u are each a natural number. The sum of r, s, t and u is from 5 to 30. R²⁴ is a C₁₆₋₂₄ linear acyl group or linear alkyl group.

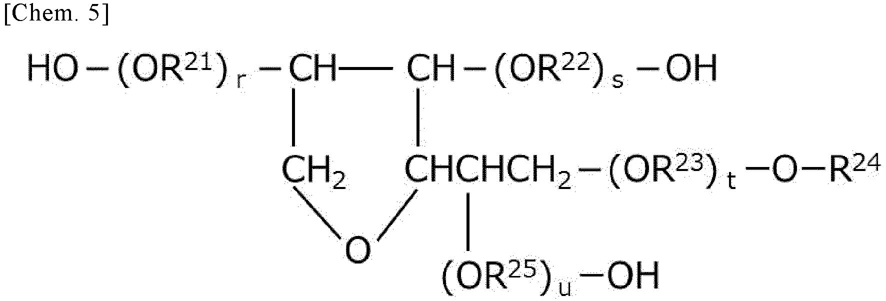

According to a prefered mode of the aforementioned first aspect, the lamellar gel phase has a higher eutectic point and enthalpy of fusion than a lamellar gel phase formed singly by any one of the second nonionic surfactants represented by the respective formulas of Chem. 3 to Chem. 5.

According to a prefered mode of the aforementioned first aspect, the content of the second nonionic surfactant is from 0.5 to 6 parts by mass relative to 1 part by mass of the first nonionic surfactant.

In the following description, PEG is an abbreviation of polyethylene glycol, POE is an abbreviation of polyoxyethylene, and POP is an abbreviation of polyoxypropylene. The number in parentheses after PEG, POE or POP indicates the average number of moles of PEG, POE groups or POP groups added in the compound in question.

In the present disclosure, "substantial amount" refers to an amount capable of bringing about effects due to addition of the compound in question.

A lamellar gel-containing composition according to a first embodiment of the present disclosure will be described. The composition according to the first embodiment includes a lamellar gel phase. The composition contains a first nonionic surfactant. The nonionic surfactant constitutes at least a portion of the lamellar gel phase.

In the present disclosure, "lamellar gel phase" refers to a gel substance constituted by an assembly of a lamellar bilayer membrane formed by a hydrophilic surfactant in the coexistence of water. Note, however, that generally speaking, a gel that is an assembly formed by a higher aliphatic alcohol and a hydrophilic surfactant in water and that has an α-structure (Shoji FUKUSHIMA, "Physical Chemistry of Cetyl Alcohol", Fragrance Journal Ltd.) is called a "lamellar gel".

Formation of a lamellar gel phase can be verified by X-ray scattering pattern analysis. For example, in cases where a plurality of peaks corresponding to the long spacing are found in the small-angle region and a single sharp peak is found in the wide-angle region (scattering vector q = 1.5 nm⁻¹), it can be determined that a lamellar gel phase has been formed.

### First Nonionic Surfactant:

The lamellar gel-containing composition of the present disclosure contains a first nonionic surfactant which is considered to constitute at least a portion of the lamellar gel phase. The first nonionic surfactant is preferably a nonionic surfactant having hydrophobic moieties respectively on both sides of a hydrophilic moiety. For example, the first nonionic surfactant may be a nonionic surfactant having a structure as shown in Chem. 6 below. In the formula shown in Chem. 6, R¹ is a C₁₆₋₂₄ linear acyl group or linear alkyl group. R² is a C₂₋₄ alkylene group. R³ is a C₁₆₋₂₄ linear acyl group or linear alkyl group. The letter k is an integer from 4 to 15.

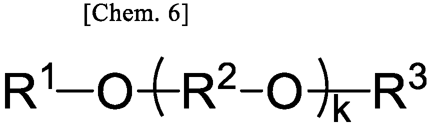

Examples of the first nonionic surfactant may include polyoxyethylene (4 mol) distearate (e.g., Emalex 200DIS from Nihon Emulsion Co., Ltd.), polyoxyethylene (6 mol) distearate (e.g., Emalex 300DIS from Nihon Emulsion Co., Ltd.), polyoxyethylene (8 mol) distearate (e.g., Emalex 400DIS from Nihon Emulsion Co., Ltd.), polyoxyethylene (12 mol) distearate (e.g., Emalex 600DIS from Nihon Emulsion Co., Ltd.), steareth-4 stearate (e.g., Emalex SWS-4 from Nihon Emulsion Co., Ltd.), steareth-6 stearate (e.g., Emalex SWS-6 from Nihon Emulsion Co., Ltd.), steareth-9 stearate (e.g., Emalex SWS-9 from Nihon Emulsion Co., Ltd.), polyoxyethylene (8 mol) dibehenyl ether, etc. Note that the bonding form between the polyoxyethylene chain and the alkyl group(s) may be an ester or an ether, or may include both.

The content by percentage of the first nonionic surfactant relative to the mass of the composition is preferably 0.05% by mass or greater. The content by percentage of the first nonionic surfactant may be 0.1 % by mass or greater, 0.5% by mass or greater, 1% by mass or greater, 2% by mass or greater, 5% by mass or greater, 10% by mass or greater, 15% by mass or greater, 20% by mass or greater, or 25% by mass or greater. If the content by percentage of the first nonionic surfactant is less than 0.05% by mass, it becomes difficult to form a gel that has stability with respect to polar oils. The content by percentage of the first nonionic surfactant relative to the mass of the composition is preferably 40% by mass or less. The content by percentage of the first nonionic surfactant may be 30% by mass or less, 25% by mass or less, 20% by mass or less, 15% by mass or less, 10% by mass or less, or 5% by mass or less. If the content by percentage of the first surfactant exceeds 40% by mass, the viscosity will become extremely high, thus impairing the feel upon use.

The lamellar gel-containing composition according to the first embodiment of the present disclosure further contain a second nonionic surfactant. The second nonionic surfactant is considered to constitute at least a portion of the lamellar gel phase together with the first nonionic surfactant. The second nonionic surfactant has an HLB of 7 or greater. The second nonionic surfactant has an HLB of 15 or less.

The second nonionic surfactant may be at least one nonionic surfactant selected from the group consisting of compounds represented by the formulas shown in Chem. 8 to Chem. 10 below. A plurality of types of second nonionic surfactants may be used in combination.

In the formula shown in Chem. 8, R⁶, R¹⁰ and R¹⁴ are each a C₂₋₄ alkylene group. R⁷, R¹¹ and R¹⁵ are each a C₈₋₁₂ alkylene group. R⁸, R¹² and R¹⁶ are each a C₄₋₈ alkyl group. R⁹, R¹³ and R¹⁷ are each a polymer of 12-hydroxystearic acid or a polymer of an alkylene polyol. The degree of polymerization of the polymer of 12-hydroxystearic acid or the polymer of an alkylene polyol may be, for example, from 1 to 3. The letters m, n and o are each a natural number. The sum of m, n and o is from 10 to 60.

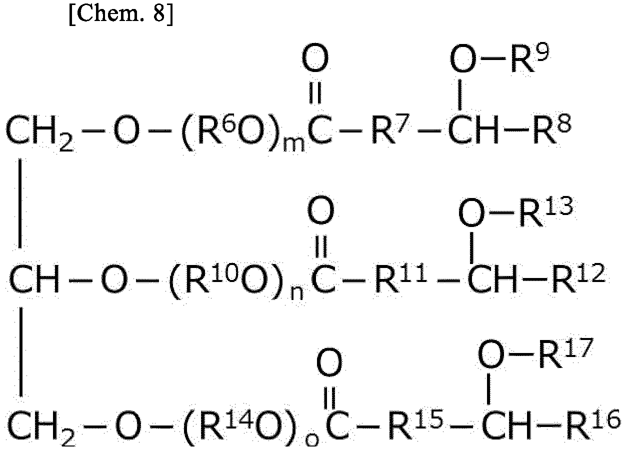

Examples of the second nonionic surfactant shown in Chem. 8 may include polyoxyethylene (20 mol) glyceryl hydrogenated castor oil (e.g., Nikkol HCO-20 from Nikko Chemicals Co., Ltd.), polyoxyethylene (60 mol) glyceryl hydrogenated castor oil (e.g., Nikkol HCO-60 from Nikko Chemicals Co., Ltd.), etc.

In the formula shown in Chem. 9, R¹⁸ is a C₁₆₋₂₄ linear acyl group or linear alkyl group. R¹⁹ and R²⁰ are each a C₂₋₄ alkylene group. The letters p and q are each a natural number. The sum of p and q is from 5 to 20.

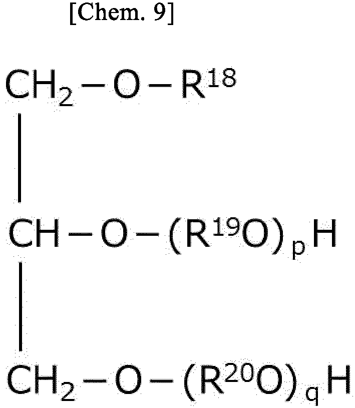

Examples of the second nonionic surfactant shown in Chem. 9 may include polyoxyethylene (5 mol) glyceryl monostearate (e.g., Emalex GM-5 from Nihon Emulsion Co., Ltd.), polyoxyethylene (10 mol) glyceryl monostearate (e.g., Emalex GM-10 from Nihon Emulsion Co., Ltd.), etc.

In the formula shown in Chem. 10, R²¹, R²², R²³ and R²⁵ are each a C₂₋₄ alkylene group. The letters r, s, t and u are each a natural number. The sum of r, s, t and u is from 5 to 30. R²⁴ is a C₁₆₋₂₄ linear acyl group or linear alkyl group.

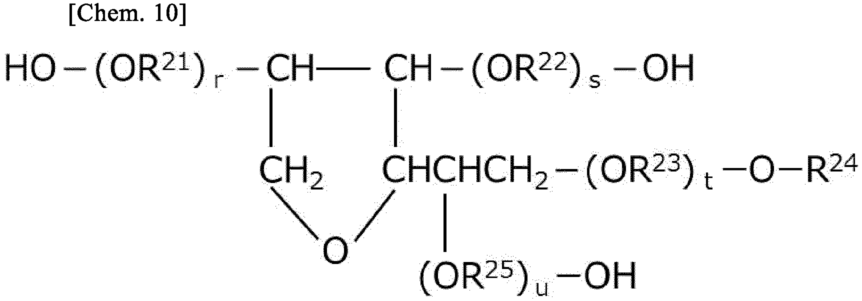

Examples of the second nonionic surfactant shown in Chem. 10 may include polyoxyethylene (20 mol) sorbitan monostearate (e.g., Nikkol TS-10V from Nikko Chemicals Co., Ltd.), etc.

The content by percentage of the second nonionic surfactant relative to the mass of the composition is preferably 0.05% by mass or greater. The content by percentage of the second nonionic surfactant may be 0.1% by mass or greater, 0.5% by mass or greater, 1% by mass or greater, 2% by mass or greater, 5% by mass or greater, 10% by mass or greater, 15% by mass or greater, 20% by mass or greater, or 25% by mass or greater. If the content by percentage of the second nonionic surfactant is less than 0.05% by mass, it becomes difficult to form a lamellar gel. The content by percentage of the second nonionic surfactant relative to the mass of the composition is preferably 60% by mass or less. The content by percentage of the second nonionic surfactant may be 50% by mass or less, 40% by mass or less, 30% by mass or less, 25% by mass or less, 20% by mass or less, 15% by mass or less, 10% by mass or less, or 5% by mass or less.

The mass ratio between the first nonionic surfactant and the second nonionic surfactant may preferably be at least 0.5 parts by mass of the second nonionic surfactant relative to 1 part by mass of the first nonionic surfactant. The ratio of the second nonionic surfactant relative to 1 part by mass of the first nonionic surfactant may be 1 part by mass or greater, 1.5 parts by mass or greater, 2 parts by mass or greater, 2.5 parts by mass or greater, or 3 parts by mass or greater. The mass ratio between the first nonionic surfactant and the second nonionic surfactant may preferably be at most 5 parts by mass of the second nonionic surfactant relative to 1 part by mass of the first nonionic surfactant. The ratio of the second nonionic surfactant relative to 1 part by mass of the first nonionic surfactant may be 4 parts by mass or less, 3 parts by mass or less, or 2 parts by mass or less. If the ratio of the second nonionic surfactant relative to 1 part by mass of the first nonionic surfactant exceeds 5 parts by mass, it becomes difficult to form a lamellar gel that has high stability with respect to polar oils.

### Higher Aliphatic Alcohols and Higher Fatty Acids:

In the composition of the present disclosure, a lamellar gel phase can be constructed only by the nonionic surfactant(s). In the composition of the present disclosure, the content by percentage of higher aliphatic alcohols and/or higher fatty acids having at least 16 carbon atoms and constituting the lamellar gel phase is preferably 1% by mass or less relative to the mass of the composition. The composition of the present disclosure may include substantially no higher aliphatic alcohol or higher fatty acid having at least 16 carbon atoms. If the content by percentage of higher aliphatic alcohols and/or higher fatty acids constituting the lamellar gel phase exceeds 1% by mass, the user may feel sliminess when applying the composition to the skin.

### Water:

The lamellar gel-containing composition of the present disclosure may further contain water. Examples of water that may be used include water used in such products as cosmetics, quasi-pharmaceutical products, etc., with usable examples including purified water, ion-exchanged water, tap water, etc.

The content by percentage of water relative to the mass of the composition may be 10% by mass or greater, 15% by mass or greater, 20% by mass or greater, 25% by mass or greater, 30% by mass or greater, 35% by mass or greater, or 40% by mass or greater. The content by percentage of water relative to the mass of the composition may be 90% by mass or less, 80% by mass or less, 70% by mass or less, 60% by mass or less, 50% by mass or less, 40% by mass or less, 30% by mass or less, 25% by mass or less, or 20% by mass or less.

A method for producing the lamellar gel-containing composition according to the first embodiment of the present disclosure will be described. A method for producing the lamellar gel composition may involve, for example: a step of heating and melting the aforementioned nonionic surfactant(s); and a step of adding water to the molten nonionic surfactant(s) and stirring the same. The nonionic surfactant can be molten, for example, at 70°C to 80°C. It is preferred that the water to be added is heated to the same extent (e.g., 70°C to 80°C) as the nonionic surfactant. The lamellar gel phase can be obtained by cooling after adding water. In cases of employing a mixture of the nonionic surfactants, for example, a mixture including a nonionic surfactant as shown in Chem. 6 and a nonionic surfactant as shown in any of Chem. 8 to Chem. 10 can be heated and molten.

The lamellar gel phase of the present disclosure can be constructed from the nonionic surfactant(s) without using any higher aliphatic alcohol or higher fatty acid. In this way, when the lamellar gel phase is applied to the skin, the user can obtain a refreshing feel upon use without feeling any sliminess, which is perceived from a lamellar gel phase made using higher aliphatic alcohols or higher fatty acids. Particularly, since it is thought that not much aqueous components are incorporated between the lamellar planes, conformance to the skin during application can be accelerated, and also, excellent moisture-retentive action on the skin can be achieved after application.

The composition of the present disclosure may include a lamellar gel phase constituted as a single-phase self-assembled structure. In the composition of the present disclosure, it is possible to suppress the production of a biphase mixture consisting of the lamellar gel phase and crystal. Thus, in the composition of the present disclosure, it is possible to suppress problems related to stability, such as crystal precipitation, viscosity increase, etc.

A lamellar gel phase of the present disclosure formed by the nonionic surfactant shown in Chem. 6 and nonionic surfactant(s) shown in Chem. 8 to Chem. 10 has a higher eutectic point and enthalpy of fusion than a lamellar gel phase formed singly by the nonionic surfactant shown in Chem. 8 to Chem. 10. A higher eutectic point and enthalpy of fusion can improve stability against high temperatures.

An emulsified composition according to a second embodiment of the present disclosure will be described. The emulsified composition of the present disclosure contains: the lamellar gel phase-containing composition according to the first embodiment; an oily component; and water. The emulsified composition may be a water-in-oil-type emulsified composition in which water is emulsified by the lamellar gel phase. Alternatively, the emulsified composition may be an oil-in-water-type composition in which an oily component is emulsified by the lamellar gel phase.

The oily component is not particularly limited, and for example, any of various components, such as liquid oils, solid fats, waxes, hydrocarbon oils, higher fatty acids, higher alcohols, synthetic ester oils, silicone oils, etc., may be blended as appropriate.

Examples of the liquid oil that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil, and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils that may be used may include liquid paraffin, ozocerite, squalane, pristane, paraffin, ceresin. squalene, vaseline, microcrystalline wax, and the like.

Examples of the higher fatty asid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid (EPA), docosahexaenoic acid (DHA) and the like.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber, cyclopentasiloxane and the like.

The content by percentage of the oily component relative to the mass of the emulsified composition is preferably 0.05% by mass or greater. The content by percentage of the oily component may be, for example, 1% by mass or greater, 5% by mass or greater, 10% by mass or greater, 15% by mass or greater, 20% by mass or greater, 25% by mass or greater, or 30% by mass or greater. If the content of the oily component is less than 0.05% by mass, the effects of using the composition as an external-use skin preparation will deteriorate. The content by percentage of the oily component may be, for example, 45% by mass or less, 40% by mass or less, 35% by mass or less, 30% by mass or less, 25% by mass or less, 20% by mass or less, or 15% by mass or less. The content by percentage of the oily component relative to the mass of the emulsified composition is preferably 50% by mass or less. If the content of the oily component exceeds 50% by mass, the feel upon use on the skin will deteriorate.

In the second embodiment, at least 1% by mass of higher aliphatic alcohol(s) and/or higher fatty acid(s) may be included as the oily component.

In the second embodiment, the respective contents by percentage of the first nonionic surfactant, the second nonionic surfactant and water can be calculated from the content by percentage of the lamellar gel phase-containing composition described below. Further, as regards the respective contents by percentage of the first nonionic surfactant, the second nonionic surfactant and water in the second embodiment, the description in the foregoing first embodiment may be incorporated by reference, and detailed description thereon is omitted herein.

A method for producing the emulsified composition according to the second embodiment of the present disclosure will be described below. As a first mode, a method for producing an oil-in-water-type composition may involve, for example: a step of emulsifying an oily component by the lamellar gel phase; and a step of adding an aqueous component after the emulsification. The step of emulsifying the oily component may involve, for example: a step of preparing a solution in which the first nonionic surfactant and the second nonionic surfactant are dissolved in a polyol (e.g., dipropylene glycol and/or 1,3-butylene glycol); and a step of emulsifying the oily component while adding the oily component to the solution. For example, the emulsified composition of the present disclosure may be produced by employing nonaqueous emulsification (D-phase emulsification method). By employing nonaqueous emulsification, the emulsion particles can be made even finer.

As a second mode, a method for producing the emulsified composition of the present disclosure may involve, for example: a step of preparing the composition according to the first embodiment; and a step of emulsifying an oily component or water by the lamellar gel phase in the composition.

In the emulsified composition according to the second embodiment, an arbitrary amount of the lamellar gel phase-containing composition according to the first embodiment may be blended. For example, in the emulsified composition, the lamellar gel-containing composition may be blended such that the total amount of nonionic surfactant(s) forming the lamellar gel phase is 0.1% by mass or greater relative to the mass of the emulsified composition. Further, the lamellar gel phase-containing composition may be blended such that the total amount of nonionic surfactant(s) forming the lamellar gel phase is 20% by mass or less relative to the mass of the emulsified composition.

The emulsified composition of the present disclosure has higher stability and quicker skin conformability compared to emulsions obtained by emulsification with a lamellar gel phase employing higher aliphatic alcohol(s) and/or higher fatty acid(s).

The lamellar gel phase of the present disclosure has low solubility with respect to highly polar oily components. Thus, it is possible to emulsify even highly polar oils, which cannot be emulsified by a lamellar gel phase employing higher aliphatic alcohol(s) and/or higher fatty acid(s).

A composition for an external-use skin preparation according to a third embodiment of the present disclosure will be described. The composition for an external-use skin preparation of the present disclosure contains at least one selected from the lamellar gel phase-containing composition according to the first embodiment and the emulsified composition according to the second embodiment. Stated differently, the lamellar gel phase-containing composition according to the first embodiment, as well as the emulsified composition according to the second embodiment, can be used as an external-use skin preparation.

If necessary, the lamellar gel phase-containing composition, the emulsified composition and the composition for an external-use skin preparation of the present disclosure may contain other components as appropriate, such as water-soluble alcohols, powders, anionic surfactants, cationic surfactants, amphoteric surfactants, hydrophilic nonionic surfactants, oleophilic nonionic surfactants, water-soluble polymers, thickeners, moisturizers, film-forming agents, oil-soluble UV absorbers, water-soluble UV absorbers, metal ion sequestering agents, amino acids, organic amines, polymer emulsions, pH adjusters, skin nutrients, vitamins, antioxidants, antioxidant aids, perfumes, etc., in amounts that do not inhibit the effects of the present disclosure.

Examples of water-soluble alcohols may include at least one type selected from lower alcohols, polyhydric alcohols, polyhydric alcohol polymers, dihydric alcohol alkyl ethers, dihydric alcohol alkyl ethers, dihydric alcohol ether esters, glycerin monoalkyl ethers, sugar alcohols, monosaccharides, oligosaccharides, polysaccharides, and derivatives of the above.

Examples of the lower alcohol may include ethanol, propanol, isopropanol, isobutyl alcohol, t-butyl alcohol, and the like.

Examples of the dihydric alcohol may include ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycolhol, and the like.

Examples of the trihydric alcohol may include glycerin, trimethylolpropane, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heptulose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

The powder is not particularly limited so long as it is generally usable for cosmetic purposes, for example. Examples of the powder may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc); wax powder (such as carnauba wax powder, etc.); starch powder (such as cornstarch powder, rice starch powder, etc) and the like.

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); dialkyldimethyl ammonium salt (such as distearyldimethyl ammonium chloride); poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, and the like.

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,000, etc); acrylic polymer (such as sodium polyacrylate, polyethylacrylate, polyacrylamide, etc); polyethyleneimine; cationic polymer; and the like.

Examples of the thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride, taurate-based synthetic polymers, and acrylate-based synthetic polymers.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract, and the like.

Examples of the film-forming agent may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc), and the like.

Examples of oil-soluble UV absorbers may include: benzoic acid-based UV absorbers (e.g., para-aminobenzoic acid (abbreviated as PABA hereinbelow), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc.); anthranilic acid-based UV absorbers (e.g., homomenthyl-N-acetylanthranilate, etc.); salicylic acid-based UV absorbers (e.g., amyl salicylate, menthyl salicylate, homomenthyl salicylate, octyl salicylate, phenyl salicylate, benzyl salicylate, p-isopropanol phenyl salicylate, homosalate, etc.); cinnamic acid-based UV absorbers (e.g., octyl methoxycinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate, ethylhexyl methoxycinnamate), 2-ethoxyethyl-p-methoxycinnamate, cyclohexyl-p-methoxycinnamate, ethyl-α-cyano-β-phenyl cinnamate, 2-ethylhexyl-α-cyano-β-phenyl cinnamate, glyceryl mono-2-ethylhexanoyl-di-para-methoxycinnamate, etc.); 3-(4'-methylbenzylidene)-d,l-camphor, 3-benzylidene-d,l-camphor; 2-phenyl-5-methylbenzoxazole; 2,2'-hydroxy-5-methylphenyl benzotriazole; 2-(2'-hydroxy-5'-t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazinone; 2-ethylhexyl-2-cyano-3,3-diphenyl acrylate (octocrylene); 2,4-bis-{[4-(2-ethylhexyloxy)-2-hydroxy]-phenyl}-6-(4-methoxyphenyl)-(1,3,5)-tri azine; and benzophenone-based UV absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid salt, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc.).

Examples of water-soluble UV absorbers may include: benzophenone-based UV absorbers (e.g. 2-hydroxy-4-methoxybenzophenone-5-sulfate, etc.); benzylidene camphor-based UV absorbers (e.g. benzylidene camphor sulfonic acid, terephthalylidene dicamphor sulfonic acid, etc.); phenylbenzimidazole-based UV absorbers (e.g. phenylbenzimidazole sulfonic acid, etc.)

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

Examples of the pH modifier may include buffer such as lactic acid-sodium lactate, citric acid-sodium citrate, succinic acid-sodium succinate, and the like.

Examples of the vitamins may include vitamine A, B1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure further may inculde, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

### Examples

The lamellar gel phase-containing composition and the emulsified composition of the present disclosure will be described below by way of examples. The lamellar gel phase-containing composition and the emulsified composition of the present disclosure are, however, not limited to the following examples. The content by percentage of each of the components shown in the Tables is in terms of percent by mass (mass%).

### Test Examples 1 to 4:

Compositions shown in Tables 1 to 4 were prepared, and each composition was subjected to small angle/wide angle X-ray scattering measurement and differential scanning calorimetry measurement (DSC measurement). The number of moles in parentheses shown in Tables 1 to 4 indicates the average number of moles of polyoxyethylene added.

Polyoxyethylene (6 mol) distearate was used as the first nonionic surfactant, which was a double-chained nonionic surfactant. As for the second nonionic surfactant having an HLB of from 7 to 15, the following components were used, respectively: polyoxyethylene (7 mol) cetyl ether (HLB: 10); a 3:2 mixture of polyoxyethylene (20 mol) glyceryl hydrogenated castor oil (HLB: 10.5) and polyoxyethylene (10 mol) glyceryl hydrogenated castor oil (HLB: 7); polyoxyethylene (10 mol) glyceryl monostearate (HLB: 11); and polyoxyethylene (20 mol) sorbitan monostearate (HLB: 14.9).

Each composition of the present disclosure was prepared by melting the nonionic surfactants shown in the Tables below at a temperature of from 70°C to 80°C into a single phase, adding thereto ion-exchanged water having a temperature of 70°C to 80°C, stirring the mixture, and then cooling the same.

Small angle/wide angle X-ray scattering was measured with respect to a 25°C lamellar gel. As for differential scanning calorimetry, the change in heat quantity was measured for when the temperature was raised from 30°C to 70°C at a rate of 2°C per minute (DSC measurement). A sample to which the first nonionic surfactant was added and a sample to which the first nonionic surfactant was not added were compared.

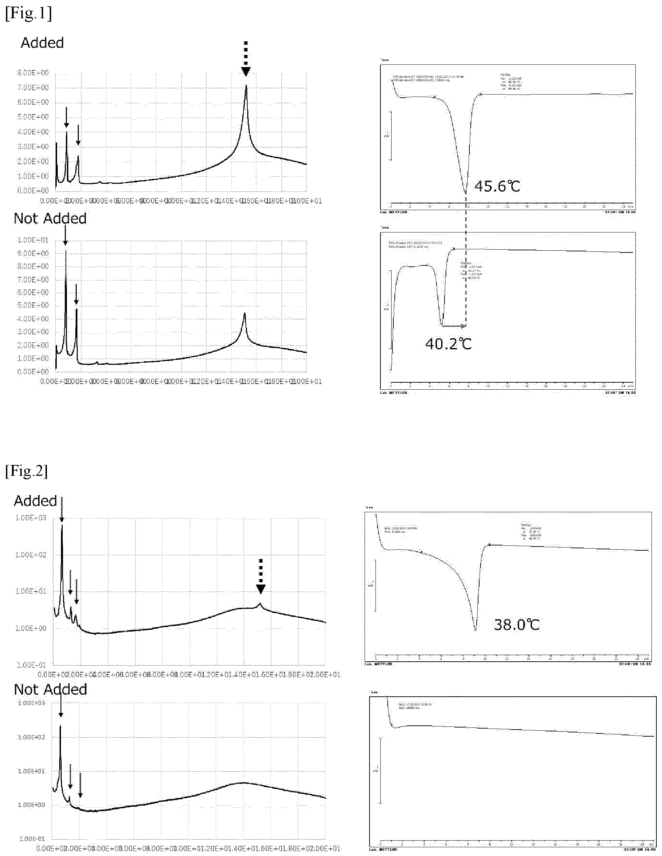

FIGS. 1 to 4 respectively show small angle/wide angle X-ray scattering charts and DSC charts for Test Examples 1 to 4. In each Figure, the charts on the upper side (indicated as "Added") are for the composition to which the first nonionic surfactant has been added, whereas the charts on the lower side (indicated as "Not Added") are for the composition to which the first nonionic surfactant has not been added.

In Test Example 1 (FIG. 1), the arrows appearing at regular intervals at a ratio of 1:2 on the small-angle side of the X-ray chart correspond to the long spacing of the lamellar gel, showing that both the sample to which polyoxyethylene (6 mol) distearate was added, as well as the sample to which the same was not added, had a lamellar structure. In contrast, the peak near the scattering vector q = 1.5 nm⁻¹, shown by the large dotted arrow, indicates an α-structure hexagonal crystal lattice; the increase in intensity of this peak shows that the regularity of the sublattice plane has increased. As for DSC, adding polyoxyethylene (6 mol) distearate caused the endothermic peak-which was 40.2°C in the Not Added sample-to shift to 45.6°C, and also caused the original peak to disappear. It was thus found that this peak is the eutectic point of the mixture.

In Test Example 2 (FIG. 2), the Not Added sample had no melting point, nor had a peak near the scattering vector q = 1.5 nm⁻¹, showing that the sample is a lamellar liquid crystal that does not have a hexagonal crystal structure. However, adding polyoxyethylene (6 mol) distearate caused a hexagonal crystal peak to appear and caused a eutectic point to appear at 38.0°C.

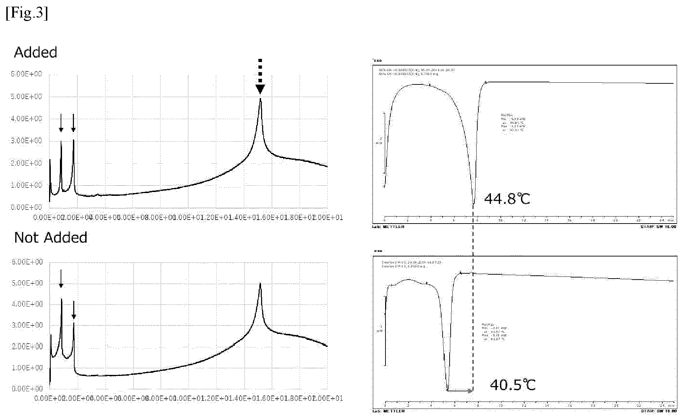

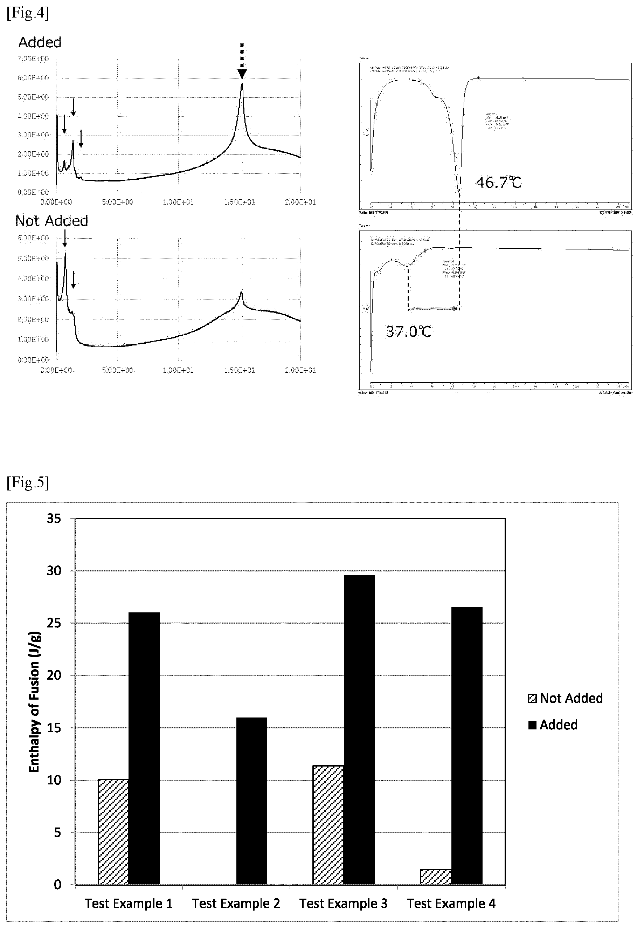

Also in Test Example 3 (FIG. 3) and Test Example 4 (FIG. 4), adding polyoxyethylene (6 mol) distearate increased the regularity of the sublattice plane and also caused a eutectic point to appear at a point approximately 5°C higher than the original melting point.

The above results show that, in each of the compositions of the present disclosure, the first nonionic surfactant, the second nonionic surfactant and water formed a single-phase self-assembled structure. For example, in case of a two-phase mixture consisting of crystal and lamellar gel, lumps of crystal will precipitate in the composition. In contrast, it was shown that the compositions of the present disclosure were uniform. It was also shown that the compositions of the present disclosure had excellent high-temperature stability.

FIG. 5 shows comparisons between the enthalpy of fusion ("Added") of compositions each containing a mixture of the first nonionic surfactant and the second nonionic surfactant of Test Examples 1 to 4, and the enthalpy of fusion ("Not Added") of compositions each containing a lamellar gel or lamellar liquid crystal constituted singly by the respective second nonionic surfactant.

As shown in FIG. 5, adding polyoxyethylene (6 mol) distearate caused a significant increase in the enthalpy of fusion of each self-assembled structure of the nonionic surfactant aqueous dispersion. This shows that adding polyoxyethylene (6 mol) distearate increases the regularity of the self-assembled structure of the nonionic surfactants, thereby making the lamellar gel firmer.

**[Table 1] (reference examples)**

| Test Example | 1-1 | 1-2 |
|---|---|---|
| Polyoxyethylene (6 mol) distearate *¹ | 20 | - |
| Polyoxyethylene (7 mol) cetyl ether *² | 30 | 50 |
| Ion-exchanged water | 50 | 50 |
| Total | 100 | 100 |
| Enthalpy of fusion (J/g) | 26.0 | 10.1 |
| *1: Emalex 300DIS (from Nihon Emulsion Co., Ltd.) | | |
| *2: Emalex 107 (from Nihon Emulsion Co., Ltd.) | | |

**[Table 2]**

| Test Example | 2-1 | 2-2 |
|---|---|---|
| Polyoxyethylene (6 mol) distearate *¹ | 30.8 | - |
| Polyoxyethylene (20 mol) glyceryl hydrogenated castor oil *³ | 27.7 | 46.1 |
| Polyoxyethylene (10 mol) glyceryl hydrogenated castor oil *⁴ | 18.4 | 30.8 |
| Ion-exchanged water | 23.1 | 23.1 |
| Total | 100 | 100 |
| Enthalpy of fusion (J/g) | 16.0 | 0 |
| *3: Nikkol HCO-20 (from Nikko Chemicals Co., Ltd.) | | |
| *4: Emalex HC⁻10 (from Nihon Emulsion Co., Ltd.) | | |

**[Table 3]**

| Test Example | 3-1 | 3-2 |
|---|---|---|
| Polyoxyethylene (6 mol) distearate *¹ | 20 | - |
| Polyoxyethylene (10 mol) glyceryl monostearate *⁵ | 30 | 50 |
| Ion-exchanged water | 50 | 50 |
| Total | 100 | 100 |
| Enthalpy of fusion (J/g) | 29.6 | 11.4 |
| *5: Emalex GM-10 (from Nihon Emulsion Co., Ltd.) | | |

**[Table 4]**

| Test Example | 4-1 | 4-2 |
|---|---|---|
| Polyoxyethylene (6 mol) distearate *¹ | 25 | - |
| Polyoxyethylene (20 mol) sorbitan monostearate *⁶ | 25 | 50 |
| Ion-exchanged water | 50 | 50 |
| Total | 100 | 100 |
| Enthalpy of fusion (J/g) | 26.5 | 1.5 |
| *6: Nikkol TS-10V (from Nikko Chemicals Co., Ltd.) | | |

### Test Example 5:

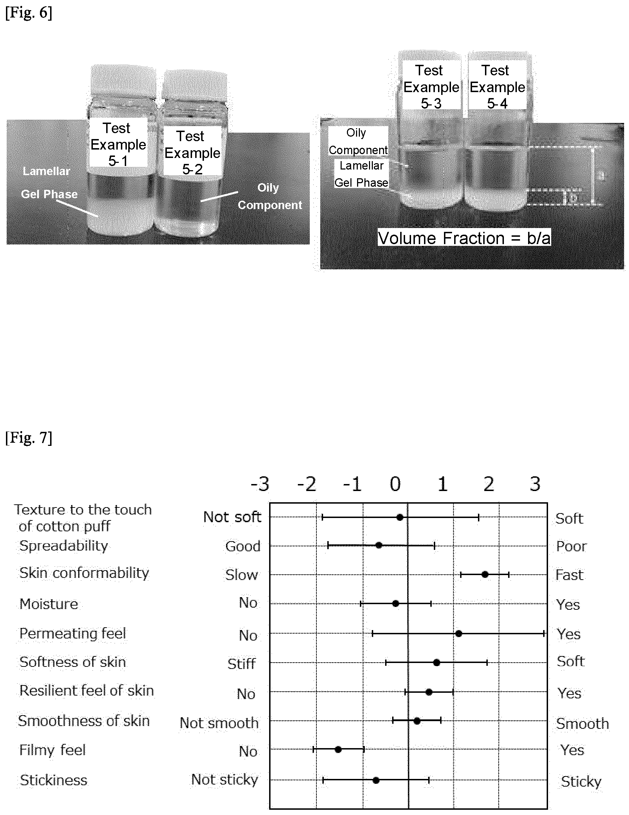

The solubility of the various lamellar gel phases of the present disclosure with respect to polar oily components was tested. Compositions each including the respective lamellar gel phase with the components shown in Table 5 were prepared. To each composition, an equivalent mass of tripropylene glycol dipivalate (Salacos TPG from The Nisshin OilliO Group, Ltd.), which is a highly polar oily component, was added, and the mixture was stored in a thermoregulated oven at 50°C for 1 month, to verify the solubility of each lamellar gel with respect to tripropylene glycol dipivalate. The solubility was compared in terms of volume fraction of the lamellar gel phase. The "volume fraction" is a value found by multiplying, by 100, the ratio (b/a) of the height (b) from the bottom surface to the upper surface of the lamellar gel phase (whitish portion) to the height (a) from the bottom surface to the liquid surface. FIG. 6 shows photographs of the respective compositions of Test Examples 5-1 to 5-4. The compositions of Test Example 5-1 and Test Example 5-2 are the same as the compositions of Test Example 3-1 and Test Example 3-2.

In the composition of Test Example 5-2 which did not include the first nonionic surfactant, the lamellar gel dissolved into the oily component. As for Test Examples 5-3 and 5-4 in which the lamellar gel phase was formed by adding a higher alcohol, there was some lamellar gel phase that remained without dissolving into the polar oil component, but the amount that dissolved was greater than the lamellar gel phase of the present disclosure shown in Test Example 5-1. This result shows that the lamellar gel phase of the present disclosure has low solubility to highly polar oily components.

**[Table 5]**

| Test Example | 5-1 | 5-2 | 5-3 | 5-4 |
|---|---|---|---|---|
| Polyoxyethylene (6 mol) distearate *¹ | 20 | - | - | - |
| Polyoxyethylene (10 mol) glyceryl monostearate *⁵ | 30 | 50 | 30 | 30 |
| Behenyl alcohol | - | - | 20 | - |
| Stearyl alcohol | - | - | - | 20 |
| Ion-exchanged water | 50 | 50 | 50 | 50 |
| Total | 100 | 100 | 100 | 100 |
| Volume fraction of lamellar gel phase (%) | 49 | 0 | 32 | 21 |

### Test Example 6:

The emulsion stability of emulsions employing the various lamellar gel phases of the present disclosure was tested. Lamellar gel phases including the components shown in Table 6 were prepared, and then, tripropylene glycol dipivalate was emulsified. The emulsified composition was stored at 50°C for 1 month, to observe whether the emulsified oil droplets became unified or not.

In Test Examples 6-5 to 6-7 which did not employ the first nonionic surfactant, unification of the emulsified oil droplets was observed. In contrast, in Test Examples 6-1 to 6-4 which each employed the lamellar gel phase of the present disclosure, unification of the emulsified oil droplets was not observed. The results of Test Examples 1 to 5 show that the lamellar gel phase of the present disclosure has an even firmer structure, and thus hardly dissolves in tripropylene glycol dipivalate. It is hence though that, since the lamellar gel phase is not incorporated into the oil droplets, unification of the emulsified particles does not occur. In contrast, in Test Examples 6-5 to 6-7, it is thought that the lamellar gel phase dissolved into the oil droplets and the oil droplets thus got unified.

**[Table 6]**

| Test Example | 6-1 | 6-2 | 6-3 | 6-4 | 6-5 | 6-6 | 6-7 |
|---|---|---|---|---|---|---|---|
| Polyoxyethylene (6 mol) distearate *¹ | 2 | 2 | 2 | 2 | - | - | - |
| Polyoxyethylene (10 mol) glyceryl monostearate *⁵ | 3 | - | - | - | 5 | 3 | 3 |
| Polyoxyethylene (7 mol) cetyl ether *² | - | 3 | - | - | - | - | - |
| Polyoxyethylene (20 mol) glyceryl hydrogenated castor oil *³ | - | - | 2 | - | - | - | - |
| Polyoxyethylene (10 mol) glyceryl hydrogenated castor oil *⁴ | - | - | 1 | - | - | - | - |
| Polyoxyethylene (20 mol) sorbitan monostearate *⁶ | - | - | - | 3 | - | - | - |
| Behenyl alcohol | - | - | - | - | - | 2 | - |
| Stearyl alcohol | - | - | - | - | - | | 2 |
| Ion-exchanged water | 75 | 75 | 75 | 75 | 75 | 75 | 75 |
| Tripropylene glycol dipivalate | 20 | 20 | 20 | 20 | 20 | 20 | 20 |
| Total | 100 | 100 | 100 | 100 | 100 | 100 | 100 |
| Unification of oil droplets | No | No | No | No | Yes | Yes | Yes |

### Test Example 7:

The feel upon use of oil-in-water (O/W) type emulsified external-use skin preparations employing the various lamellar gel phases of the present disclosure was tested. Test Examples 7-1 and 7-2 are moisturizers each employing the lamellar gel phase of the present disclosure. Test Example 7-3, used as a control for comparison, is a lamellar gel phase-containing moisturizer employing behenic acid soap, stearic acid soap, polyoxyethylene (5 mol) glyceryl monostearate, glyceryl monostearate and behenyl alcohol.

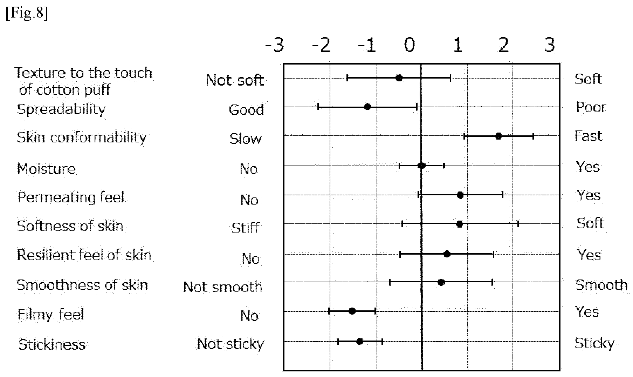

An expert panelist applied the composition of Test Example 7-1 to half of his/her face, and applied the composition of Test Example 7-3 to the remaining half of his/her face, and evaluated the feel upon use of the composition of Test Example 7-1 relative to the composition of Test Example 7-3 in terms of the evaluation items shown in FIG. 7 on a 7-level scale, ranging from -3 to +3. Likewise, the feel upon use of the composition of Test Example 7-2 relative to the composition of Test Example 7-3 was evaluated. FIG. 7 shows the evaluation results for Test Example 7-1. FIG. 8 shows the evaluation results for Test Example 7-2. The evaluation score "0 (zero)" means that the feel upon use was the same as Test Example 7-3.

Compared to the moisturizer employing the lamellar gel phase of Test Example 7-3, the moisturizers employing the respective lamellar gel phases of the present disclosure had good spreadability and permeating feel, and also offered excellent softness and resilience to the skin after application despite having a refreshing feel. The results of FIGS. 7 and 8 show that the lamellar gel phases of the present disclosure had a better feel upon use than the lamellar gel phase of Test

### Example 7-3.

**[Table 7]**

| Test Example | | 7-1 | 7-2 | 7-3 |
|---|---|---|---|---|
| (1) | Polyoxyethylene (6 mol) distearate | 0.3 | 0.3 | - |
| (2) | Polyoxyethylene (10 mol) phytosterol | 0.5 | - | - |
| (3) | Polyoxyethylene (20 mol) glyceryl hydrogenated castor oil | 0.27 | - | - |
| (4) | Polyoxyethylene (10 mol) glyceryl hydrogenated castor oil | 0.18 | - | - |
| (5) | Polyoxyethylene (10 mol) glyceryl monostearate | - | 0.7 | - |
| (6) | Behenic acid | - | - | 0.14 |
| (7) | Stearic acid | - | - | 0.09 |
| (8) | Polyoxyethylene (5 mol) glyceryl monostearate | - | - | 0.49 |
| (9) | Glyceryl monostearate | - | - | 0.38 |
| (10) | Behenyl alcohol | - | - | 0.24 |
| (11) | Microcrystalline wax | 0.5 | 0.5 | 0.5 |
| (12) | Vaseline | 5.0 | 5.0 | 5.0 |
| (13) | Diisostearyl malate | 2.0 | 2.0 | 2.0 |
| (14) | Glyceryl diisostearate | 1.0 | 1.0 | 1.0 |
| (15) | Pentaerythrityl tetraethylhexanoate | 5.0 | 5.0 | 5.0 |
| (16) | Squalane | 5.0 | 5.0 | 5.0 |
| (17) | Dimethyl polysiloxane (6 cs) | 3.0 | 3.0 | 3.0 |
| (18) | Amino-modified polymer silicone/dimethyl polysiloxane (20 cs) 10% solution | 0.5 | 0.5 | 0.5 |
| (19) | Glycerin | 7.0 | 7.0 | 7.0 |
| (20) | Dipropylene glycol | 5.0 | 5.0 | 5.0 |
| (21) | 1,3-Butylene glycol | 7.0 | 7.0 | 7.0 |
| (22) | Mabit | 2.0 | 2.0 | 2.0 |
| (23) | PEG 1000 | 1.0 | 1.0 | 1.0 |
| (24) | General alcohol, 95% | 5.0 | 5.0 | 5.0 |
| (25) | Phenoxyethanol | 0.5 | 0.5 | 0.5 |
| (26) | Carboxyvinyl polymer | 0.015 | 0.015 | 0.015 |
| (27) | Caustic potash | 0.63 | 0.63 | 0.63 |
| (28) | EDTA2Na·2H₂O | 0.03 | 0.03 | 0.03 |
| (29) | Ion-exchanged water | Balance | Balance | Balance |
| Total | | 100 | 100 | 100 |

Formulation examples of the composition of the present disclosure are described below. The application examples of the composition of the present disclosure, however, are not limited by the following formulation examples. The content by percentage of each of the components shown in the Tables is in terms of percent by mass (mass%).

### Formulation Example 1: Moisturizer (Table 8) (reference example)

A moisturizer as shown in Table 8 was prepared by emulsification according to an ordinary method. The obtained moisturizer quickly conformed to the skin, had a refreshing feel, and had excellent stability.

**[Table 8]**

| Formulation Example | | 1 |
|---|---|---|
| (1) | Polyoxyethylene (6 mol) distearate | 0.3 |
| (2) | Polyoxyethylene (7 mol) cetyl ether | 0.7 |
| (3) | Dipropylene glycol | 5.0 |
| (4) | Perfume | 0.1 |
| (5) | Pentaerythrite tetra-2-ethylhexanoate | 2.0 |
| (6) | α-olefin oligomer | 3.0 |
| (7) | Dimethyl polysiloxane *⁷ | 2.0 |
| (8) | Purified vaseline | 1.0 |
| (9) | 1,3-Butylene glycol | 2.0 |
| (10) | Phenoxyethanol | 0.5 |
| (11) | Glycerin | 4.0 |
| (12) | Carboxyvinyl polymer | 0.03 |
| (13) | Potassium hydroxide | 0.01 |
| (14) | Tranexamic acid | 0.1 |
| (15) | Citric acid | 0.02 |
| (16) | Sodium citrate | 0.08 |
| (17) | Ion-exchanged water | Balance |
| Total | | 100 |
| *7: Silicone KF96-A6T (from Shin-Etsu Chemical Co., Ltd.) | | |

### Formulation Example 2: Serum (Table 9) (reference example)

A serum as shown in Table 9 was obtained by emulsification according to an ordinary method. The obtained serum quickly conformed to the skin, had a refreshing feel, and had excellent stability.

**[Table 9]**

| Formulation Example | | 2 |
|---|---|---|
| (1) | Sodium N-stearoyl methyl taurate | 0.01 |
| (2) | Polyoxyethylene (8 mol) distearate | 0.2 |
| (3) | Polyoxyethylene (10 mol) behenyl ether | 0.2 |
| (4) | Liquid paraffin | 0.78 |
| (5) | Methylphenyl polysiloxane *⁸ | 0.2 |
| (6) | Perfume | 0.02 |
| (7) | Polyoxyethylene (14 mol) polyoxypropylene (7 mol) dimethyl ether | 0.5 |
| (8) | Glycerin | 3.0 |
| (9) | Dipropylene glycol | 5.0 |
| (10) | 1,3-Butylene glycol | 3.0 |
| (11) | Citric acid | 0.02 |
| (12) | Sodium citrate | 0.08 |
| (13) | EDTA2Na·2H₂O | 0.01 |
| (14) | General alcohol, 95% | 5.0 |
| (15) | Phenoxyethanol | 0.5 |
| (16) | Ion-exchanged water | Balance |
| Total | 100 | |
| *8: Silicone KF56 (from Shin-Etsu Chemical Co., Ltd.) | | |

### Formulation Example 3: Moisturizer (Table 10)

A moisturizer as shown in Table 10 was obtained by emulsification according to an ordinary method. The obtained moisturizer quickly conformed to the skin, had a refreshing feel, and had excellent stability.

**[Table 10]**

| Formulation Example | | 3 |
|---|---|---|
| (1) | Polyoxyethylene (6 mol) distearate | 0.3 |
| (2) | Polyoxyethylene (10 mol) glyceryl monostearate | 1.0 |
| (3) | Perfume | 0.09 |
| (4) | Glyceryl tristearate | 2.5 |
| (5) | Squalane | 4.5 |
| (6) | Dimethyl polysiloxane *⁷ | 1.0 |
| (7) | Isoprene glycol | 4.5 |
| (8) | 1,4-Butanediol | 1.5 |
| (9) | Dipropylene glycol | 7.0 |
| (10) | Erythritol | 1.3 |
| (11) | Glycerin | 6.0 |
| (12) | Phenoxyethanol | 0.3 |
| (13) | Xanthan gum | 0.5 |
| (14) | Sodium hexametaphosphate | 0.03 |
| (15) | Ion-exchanged water | Balance |
| Total | | 100 |

### Formulation Example 4: Sun-Block Cream (Table 11)

A sun-block cream as shown in Table 11 was obtained by emulsification according to an ordinary method. The obtained sun-block cream quickly conformed to the skin, had a refreshing feel, and had excellent stability.

**[Table 11]**

| Formulation Example | | 4 |
|---|---|---|
| (1) | Polyoxyethylene (4 mol) distearate | 0.5 |
| (2) | Polyoxyethylene (20 mol) sorbitan monostearate | 0.5 |
| (3) | Perfume | 0.08 |
| (4) | Glyceryl tri-2-ethylhexanoate | 2.0 |
| (5) | Di-2-ethylhexyl succinate | 3.0 |
| (6) | 2-ethylhexyl p-methoxycinnamate | 5.0 |
| (7) | Avobenzone | 3.0 |
| (8) | Bis-ethylhexyloxyphenol methoxyphenyl triazine | 1.0 |
| (9) | Dipropylene glycol | 6.0 |
| (10) | 1,3-Butylene glycol | 5.0 |
| (11) | Phenoxyethanol | 0.5 |
| (12) | Glycerin | 9.0 |
| (13) | EDTA-3Na | 0.1 |
| (14) | Erythritol | 0.1 |
| (15) | Citric acid | 0.02 |
| (16) | Sodium citrate | 0.08 |
| (17) | Ion-exchanged water | Balance |
| Total | | 100 |

### Formulation Example 5: Water-in-Oil (W/O) Type Cream (Table 12)

A water-in-oil (W/O) type cream as shown in Table 12 was obtained by emulsification according to an ordinary method. The obtained water-in-oil type cream quickly conformed to the skin, had a refreshing feel, and had excellent stability.

**[Table 12]**

| Formulation Example | | 5 |
|---|---|---|
| (1) | Polyethylene glycol (6mol) dibehenate | 0.8 |
| (2) | Polyoxyethylene (5 mol) glyceryl monostearate | 0.3 |
| (3) | Perfume | 0.05 |
| (4) | Dimethyl polysiloxane *⁷ | 10.4 |
| (5) | Squalane | 5.0 |
| (6) | Behenyl alcohol | 0.6 |
| (7) | Purified vaseline | 10.0 |
| (8) | Dipropylene glycol | 5.0 |
| (9) | 1,3-Butylene glycol | 6.5 |
| (10) | Phenoxyethanol | 0.5 |
| (11) | Glycerin | 7.0 |
| (12) | EDTA-3Na | 0.1 |
| (13) | Chamomile extract | 0.1 |
| (14) | Citric acid | 0.02 |
| (15) | Sodium citrate | 0.08 |
| (16) | Ion-exchanged water | Balance |
| Total | | 100 |

The lamellar gel phase-containing compositions, emulsified compositions, and compositions for external-use skin preparations, and the methods for producing thereof according to the present invention have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

### Industrial Applicability

The lamellar gel phase-containing composition, the emulsified composition and the composition for an external-use skin preparation of the present disclosure are applicable, for example, to cosmetics applicable to the skin, cosmetics applicable to the hair, cleansers, etc. For example, the composition for an external-use skin preparation of the present disclosure is applicable, for example, to antiperspirants, deodorants, sun-block cosmetics, skin-care agents, makeup cosmetics, makeup cleansers, hair cleansers, hairdressing agents, etc.

## Claims

1. A lamellar gel phase-including composition comprising (a) a first nonionic surfactant, (b) a second nonionic surfactant having an HLB of from 7 to 15, and (c) water, wherein:
the first nonionic surfactant is represented by the following chemical formula (1): 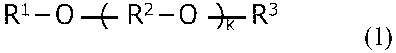 in which R¹ is a C₁₆₋₂₄ linear acyl group or linear alkyl group, R² is a C₂₋₄ alkylene group, R³ is a C₁₆₋₂₄ linear acyl group or linear alkyl group, and k represents an integer from 4 to 15;
the second nonionic surfactant is at least one selected from the group consisting of compounds represented by the following chemical formulas (3) to (5): 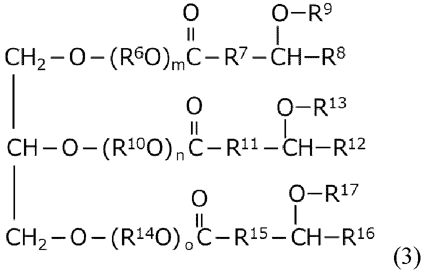
in which R⁶, R¹⁰ and R¹⁴ are each a C₂₋₄ alkylene group, R⁷, R¹¹ and R¹⁵ are each a C₈₋₁₂ alkylene group, R⁸, R¹² and R¹⁶ are each a C₄₋₈ alkyl group, R⁹, R¹³ and R¹⁷ are each a polymer of 12-hydroxystearic acid or polymer of an alkylene polyol, m, n and o are each a natural number, and the sum of m, n and o is from 10 to 60; 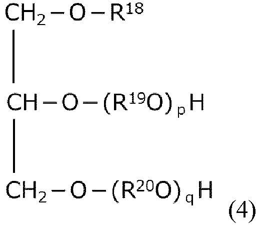
in which R¹⁸ is a C₁₆₋₂₄ linear acyl group or linear alkyl group, R¹⁹ and R²⁰ are each a C₂₋₄ alkylene group, p and q are each a natural number, and the sum of p and q is from 5 to 20; and 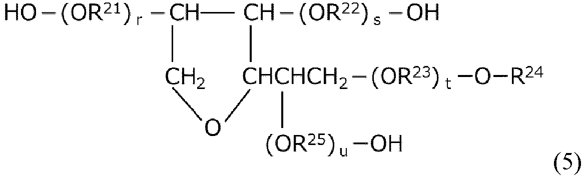
in which R²¹, R²², R²³ and R²⁵ are each a C₂₋₄ alkylene group, r, s, t and u are each a natural number, the sum of r, s, t and u is from 5 to 30, and R²⁴ is a C₁₆₋₂₄ linear acyl group or linear alkyl group;
the first nonionic surfactant, the second nonionic surfactant and the water constitute at least a portion of the lamellar gel phase; and
a content by percentage of aliphatic alcohols having at least 16 carbon atoms or fatty acids having at least 16 carbon atoms is 1% by mass or less relative to the mass of the composition.

2. The lamellar gel phase-including composition according to claim 1, wherein the first nonionic surfactant is polyethylene glycol distearate in which k shown in the chemical formula (1) is from 4 to 8.

3. The lamellar gel phase-including composition according to claim 1 or 2, wherein a content of the first nonionic surfactant is from 0.1 to 40% by mass relative to the mass of the composition.

4. The lamellar gel phase-including composition according to any one of claims 1 to 3, wherein said lamellar gel phase has a higher eutectic point and enthalpy of fusion than a lamellar gel phase formed singly by any one of the second nonionic surfactants represented by the respective chemical formulas (3) to (5).

5. The lamellar gel phase-including composition according to any one of claims 1 to 4, wherein the content of the second nonionic surfactant is from 0.5 to 6 parts by mass relative to 1 part by mass of the first nonionic surfactant.

6. An emulsified composition comprising:
the lamellar gel phase-including composition according to any one of claims 1 to 5;
an oily component; and
water,
wherein the oily component or the water is emulsified by the lamellar gel phase included in the lamellar gel phase-including composition according to any one of claims 1 to 5.

7. A composition for an external-use skin preparation, comprising the lamellar gel phase-including composition according to any one of claims 1 to 5 or the emulsified composition according to claim 6.

## Patentansprüche

1. Zusammensetzung, die eine lamellare Gelphase einschließt, umfassend (a) ein erstes nichtionisches Tensid, (b) ein zweites nichtionisches Tensid mit einem HLB-Wert von 7 bis 15 und (c) Wasser, worin:
das erste nichtionische Tensid durch die folgende chemische Formel (1) dargestellt wird: 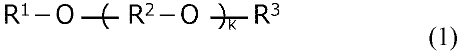
worin R¹ eine lineare C₁₆₋₂₄-Acylgruppe oder eine lineare Alkylgruppe ist, R² eine C₂₋₄-Alkylengruppe ist, R³ eine lineare C₁₆₋₂₄-Acylgruppe oder eine lineare Alkylgruppe ist und k eine ganze Zahl von 4 bis 15 darstellt;
das zweite nichtionische Tensid mindestens eines ist, ausgewählt aus der Gruppe bestehend aus Verbindungen, die durch die folgenden chemischen Formeln (3) bis (5) dargestellt werden: 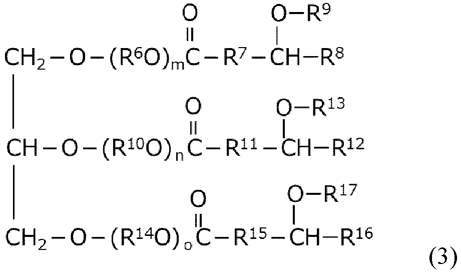
worin R⁶, R¹⁰ und R¹⁴ jeweils eine C₂₋₄-Alkylengruppe sind, R⁷, R¹¹ und R¹⁵ jeweils eine C₈₋₁₂-Alkylengruppe sind, R⁸, R¹² und R¹⁶ jeweils eine C₄₋₈-Alkylgruppe sind, R⁹, R¹³ und R¹⁷ jeweils ein Polymer von 12-Hydroxystearinsäure oder ein Polymer eines Alkylenpolyols sind, m, n und o jeweils eine natürliche Zahl sind und die Summe von m, n und o von 10 bis 60 beträgt; 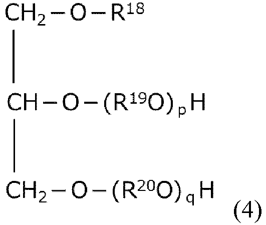
worin R¹⁸ eine lineare C₁₆₋₂₄-Acylgruppe oder eine lineare Alkylgruppe ist, R¹⁹ und R²⁰ jeweils eine C₂₋₄-Alkylengruppe sind, p und q jeweils eine natürliche Zahl sind und die Summe von p und q 5 bis 20 beträgt; und 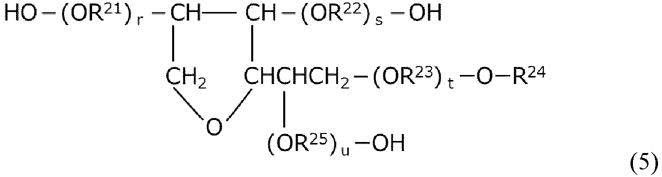
worin R²¹, R²², R²³ und R²⁵ jeweils eine C₂₋₄-Alkylengruppe sind, r, s, t und u jeweils eine natürliche Zahl sind, die Summe von r, s, t und u 5 bis 30 beträgt und R²⁴ eine lineare C₁₆₋₂₄-Acylgruppe oder lineare Alkylgruppe ist;
das erste nichtionische Tensid, das zweite nichtionische Tensid und das Wasser mindestens einen Teil der lamellaren Gelphase bilden; und
ein prozentualer Gehalt an aliphatischen Alkoholen mit mindestens 16 Kohlenstoffatomen oder Fettsäuren mit mindestens 16 Kohlenstoffatomen 1 Masse-% oder weniger, bezogen auf die Masse der Zusammensetzung, beträgt.

2. Zusammensetzung, die eine lamellare Gelphase einschließt, nach Anspruch 1, wobei das erste nichtionische Tensid Polyethylenglykoldistearat ist, bei dem k in der chemischen Formel (1) 4 bis 8 beträgt.

3. Zusammensetzung, die eine lamellare Gelphase einschließt, nach Anspruch 1 oder 2, wobei der Gehalt an dem ersten nichtionischen Tensid 0,1 bis 40 Masse-%, bezogen auf die Masse der Zusammensetzung, beträgt.

4. Zusammensetzung, die eine lamellare Gelphase einschließt, nach einem der Ansprüche 1 bis 3, wobei die lamellare Gelphase einen höheren eutektischen Punkt und eine höhere Schmelzenthalpie aufweist als eine lamellare Gelphase, die allein durch eines der zweiten nichtionischen Tenside, die durch die jeweiligen chemischen Formeln (3) bis (5) dargestellt sind, gebildet wird.

5. Zusammensetzung, die eine lamellare Gelphase einschließt, nach einem der Ansprüche 1 bis 4, wobei der Gehalt des zweiten nichtionischen Tensids 0,5 bis 6 Massenteile, bezogen auf 1 Massenteil des ersten nichtionischen Tensids, beträgt.

6. Emulgierte Zusammensetzung, umfassend:
die Zusammensetzung, die eine lamellare Gelphase einschließt, nach einem der Ansprüche 1 bis 5;
eine ölige Komponente; und
Wasser,
wobei die ölige Komponente oder das Wasser durch die lamellare Gelphase emulgiert wird, die in der die Zusammensetzung, die eine lamellare Gelphase einschließt, nach einem der Ansprüche 1 bis 5 enthalten ist.

7. Zusammensetzung für ein äußerlich anzuwendendes Hautpräparat, umfassend die Zusammensetzung, die eine lamellare Gelphase einschließt, nach einem der Ansprüche 1 bis 5 oder die emulgierte Zusammensetzung nach Anspruch 6.

## Revendications

1. Composition incluant une phase gel lamellaire comportant (a) un premier tensioactif non ionique, (b) un second tensioactif non ionique ayant un HLB de 7 à 15, et (c) de l'eau, dans laquelle :
le premier tensioactif non ionique est représenté par la formule chimique (1) suivante : 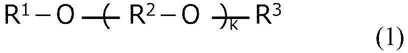
dans laquelle R¹ est un groupe acyle linéaire ou un groupe alkyle linéaire en C₁₆ à C₂₄, R² est un groupe alkylène en C₂ à C₄, R³ est un groupe acyle linéaire ou un groupe alkyle linéaire en C₁₆ à C₂₄, et k représente un entier de 4 à 15 ;
le second tensioactif non ionique est au moins un élément choisi parmi le groupe constitué de composés représentés par les formules chimiques (3) à (5) suivantes : 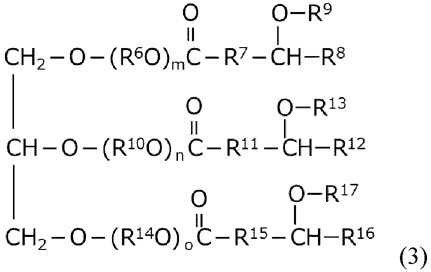
dans lesquelles R⁶, R¹⁰ et R¹⁴ sont chacun un groupe alkylène en C₂ à C₄, R⁷, R¹¹ et R¹⁵ sont chacun un groupe alkylène en C₈ à C₁₂ , R⁸, R¹² et R¹⁶ sont chacun un groupe alkyle en C₄ à C₈ , R⁹, R¹³ et R¹⁷ sont chacun un polymère d'acide 12-hydroxystéarique ou un polymère d'un alkylène polyol, m, n et o sont chacun un entier naturel, et la somme de m, n et o vaut de 10 à 60 ; 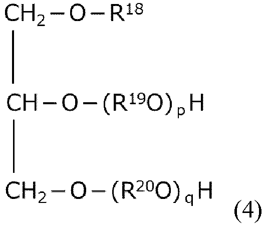
dans laquelle R¹⁸ est un groupe acyle linéaire ou un groupe alkyle linéaire en C₁₆ à C₂₄, R¹⁹ et R²⁰ sont chacun un groupe alkylène en C₂ à C₄, p et q sont chacun un entier naturel, et la somme de p et q vaut de 5 à 20 ; et 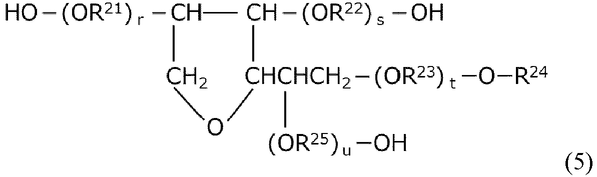
dans laquelle R²¹, R²², R²³ et R²⁵ sont chacun un groupe alkylène en C₂ à C₄ , r, s, t et u sont chacun un entier naturel, la somme de r, s, t et u vaut de 5 à 30, et R²⁴ est un groupe acyle linéaire ou un groupe alkyle linéaire en C₁₆ à C₂₄;
le premier tensioactif non ionique, le second tensioactif non ionique et l'eau constituent au moins une partie de la phase gel lamellaire ; et
une teneur en pourcentage d'alcools aliphatiques possédant au moins 16 atomes de carbone ou d'acides gras possédant au moins 16 atomes de carbone est de 1 % en masse ou moins par rapport à la masse de la composition.

2. Composition incluant une phase gel lamellaire selon la revendication 1, dans laquelle le premier tensioactif non ionique est le distéarate de polyéthylène glycol dans lequel k indiqué dans la formule chimique (1) vaut de 4 à 8.

3. Composition incluant une phase gel lamellaire selon la revendication 1 ou 2, dans laquelle une teneur en premier tensioactif non ionique est de 0,1 à 40 % en masse par rapport à la masse de la composition.

4. Composition incluant une phase gel lamellaire selon l'une quelconque des revendications 1 à 3, dans laquelle ladite phase gel lamellaire a un point eutectique et une enthalpie de fusion plus élevés qu'une phase gel lamellaire formée individuellement par l'un quelconque des seconds tensioactifs non ioniques représentés par les formules chimiques (3) à (5) respectives.

5. Composition incluant une phase gel lamellaire selon l'une quelconque des revendications 1 à 4, dans laquelle la teneur en second tensioactif non ionique est de 0,5 à 6 parties en masse par rapport à 1 partie en masse du premier tensioactif non ionique.

6. Composition émulsifiée comportant :
la composition incluant une phase gel lamellaire selon l'une quelconque des revendications 1 à 5 ;
un composant huileux ; et
de l'eau,
dans laquelle le composant huileux ou l'eau est émulsifié par la phase gel lamellaire incluse dans la composition incluant une phase gel lamellaire selon l'une quelconque des revendications 1 à 5.

7. Composition pour une préparation pour la peau à usage externe, comportant la composition incluant une phase gel lamellaire selon l'une quelconque des revendications 1 à 5 ou la composition émulsifiée selon la revendication 6.
